# EUROPEAN PATENT SPECIFICATION

(11) **EP 2 371 276 B1**
(45) Date of publication and mention of the grant of the patent: **13.01.2016**
(21) Application number: 11160138.1
(22) Date of filing: 29.03.2011
(51) Int. Cl.: A61B 5/02, A61B 5/00

(54) **Optical imaging diagnostic apparatus and the display control method thereof**
Optische Bildgebungsdiagnosevorrichtung und Anzeigesteuerungsverfahren dafür
Appareil de diagnostic d'imagerie optique et son procédé de contrôle d'affichage

(30) Priority: 30.03.2010 JP 2010078608
(43) Date of publication of application: 05.10.2011
(73) Proprietor: Terumo Kabushiki Kaisha, Tokyo 151-0072 (JP)
(72) Inventor: Onimura, Yuuji, Fujinomiya-shi Shizuoka 418-0015 (JP)
(74) Representative: Dossmann, Gérard

(56) References cited:
- EP-A1- 2 062 526
- WO-A1-2007/002969
- WO-A1-2009/121067
- US-A1- 2007 216 908

## Description

The present invention relates to an optical imaging diagnostic apparatus and a display control method thereof.

### Description of the Related Art:

From the past, there has been used an optical coherent tomography (OCT) apparatus (see, for example, Japanese unexamined patent publication No. 2001-79007) and an optical frequency domain imaging (OFDI) apparatus utilizing wavelength sweep, which is an improvement type thereof for a diagnosis of arterioscleosis, for a diagnosis before operation at the time of treatment inside a blood vessel depending on a high functional catheter such as a balloon catheter, a stent and the like or for a result confirmation after operation (Hereinafter, in the present specification, the optical coherent tomography (OCT) apparatus and the optical frequency domain imaging (OFDI) apparatus utilizing wavelength sweep will be generically referred to as "optical imaging diagnostic apparatus").

In the optical imaging diagnostic apparatus, an optical probe unit inserted with an imaging core which is attached with an optical lens and an optical mirror (transmitting and receiving unit) at a distal end of an optical fiber is inserted into a blood vessel, and a radial scan in a blood vessel is carried out by emitting a measurement light into the blood vessel from the transmitting and receiving unit at the distal end while rotating the imaging core and concurrently, by receiving a reflected light from a biological tissue. Then, a coherent light is produced by making the light-received reflected light and a reference light interfere with each other, and a cross-sectional image of the blood vessel is drawn out based on the coherent light.

Generally, on an occasion when carrying out the drawing-out of a cross-sectional image by using such an optical imaging diagnostic apparatus, it is necessary preliminarily to carry out flashing, occlusion or the like and to remove the blood inside the blood vessel for which the measurement light is emitted. It is because blood cells are strong scatterers and therefore, when there exist blood cells in the blood vessel, the measurement light attenuates considerably and it is not possible to draw out a desired cross-sectional image.

It should be noted that the wording "flashing" means that a physiological salt solution, lactate ringer, a contrast agent or the like is discharged in an area which becomes an imaging target and the blood inside the blood vessel is preliminarily removed. Also, the wording "occlusion" means that the blood vessel is blocked temporarily by a balloon or the like.

Even in a case in which flashing, occlusion or the like is carried out on one hand, it is not always true that the blood in the blood flow area inside the blood vessel can be removed perfectly. Even in such a case, if the blood quantity remaining in the blood vessel is a predetermined quantity or more, it is possible for a user to make confirmation by the drawn out cross-sectional image, but in a case in which the quantity of the blood remaining in the blood vessel are minute or in such a case in which floating thrombi are remained, it is difficult to confirm the existence of the minute bloods or thrombi in the drawn out cross-sectional image.

However, blood is a strong scatterer as mentioned above, so that even in a case in which minute bloods or thrombi exist, it happens that the measurement light attenuates in the backward area of the direction to which the measurement light is emitted. Such a phenomenon is similar to a phenomenon of attenuation of the measurement light in the backward area, which is caused by macrophage accumulated on the blood-vessel surface, and both of them are phenomena which are difficult for a user to distinguish at the time of diagnosis.

Consequently, it is difficult to confirm the existence thereof on the drawn out cross-sectional image and also, with respect to the minute bloods and thrombi (hereinafter, these are referred to as minute scatterers) which cause the attenuation of measurement light in the blood flow area inside the blood vessel, it is preferable for a user to be able to identify the presence or non-presence thereof on the cross-sectional image.

The present invention was invented in view of the problem described above and is addressed, in an optical imaging diagnostic apparatus, to make it possible for the presence or non-presence of the minute scatterer to be identified on the drawn out cross-sectional image.

An optical imaging diagnostic apparatus relating to the present invention is defined in claim 1 and also includes the following constitutions: an optical imaging diagnostic apparatus in which there is connected a probe including a transmitting and receiving unit for carrying out transmitting and receiving of light continuously, in which reflected light from a biological tissue, which the transmitting and receiving unit received, is obtained from the transmitting and receiving unit by axially moving the transmitting and receiving unit inside a body lumen while rotating it, in which a plurality of cross-sectional images of the biological tissue are generated in the axial direction by using line data with respect to coherent light produced by interference between the obtained reflected light and reference light, and the images thereof are displayed, wherein there are included:
a calculation unit for respectively calculating signal-intensity change in the circumferential direction of the circumference of the transmitting and receiving unit and signal-intensity change in the radial direction of the body lumen respectively with respect to a plurality of line data used for generation of the cross-sectional image displayed;
a specifying unit for judging, based on the calculated result in the calculation unit, whether or not there exists a scatterer which scatters the light transmitted from the transmitting and receiving unit in an blood flow area of the body lumen and for specifying the position on the displayed cross-sectional image of the scatterer in case of being judged that there exists the scatterer; and
a display unit for displaying a marker indicating that the scatterer exists at the specified position on the displayed cross-sectional image.

According to the present invention, it becomes possible in an optical imaging diagnostic apparatus to identify the presence or non-presence of a minute scatterer on a drawn out cross-sectional image.
FIG. 1 is a diagram showing an external-appearance constitution of an optical imaging diagnostic apparatus relating to one exemplified embodiment of the present invention;
FIG. 2 is a diagram showing a functional constitution in an optical coherent tomography apparatus;
FIG. 3 is a diagram showing a functional constitution in an optical frequency domain imaging apparatus utilizing wavelength sweep;
FIG. 4 is a diagram for explaining a constitution of an imaging core distal end which is inserted into an optical probe unit of an optical imaging diagnostic apparatus and an operation of an imaging core in a state of being inserted into a blood vessel;
FIGS. 5A and 5B are diagrams for explaining an imaging core operation at the time of measurement in an optical imaging diagnostic apparatus and a generation process of a blood vessel cross-sectional image;
FIG. 6 is a diagram for explaining a functional constitution of a signal processing unit in an optical imaging diagnostic apparatus;
FIG. 7A is a flowchart showing a flow of a minute scatterer detection process;
FIG. 7B is a diagram showing one example of a blood flow area on line data;
FIGS. 8A and 8B are diagrams showing one example of a user interface in a minute scatterer detection process;
FIG. 9 is a diagram for explaining a functional constitution of a signal processing unit in an optical imaging diagnostic apparatus;
FIG. 10 is a flowchart showing a flow of a minute scatterer detection process;
FIG. 11 is a diagram showing one example of a user interface in a minute scatterer detection process;
FIG. 12 is a flowchart showing a flow of a cross-sectional image correction process; and
FIG. 13 is a flowchart showing a flow of a cross-sectional image correction process.

Hereinafter, it will be explained with respect to details of respective exemplified embodiments of the present invention with reference to attached drawings.

### [First Exemplified Embodiment]

### 1. External-Appearance Constitution of Imaging Diagnostic Apparatus

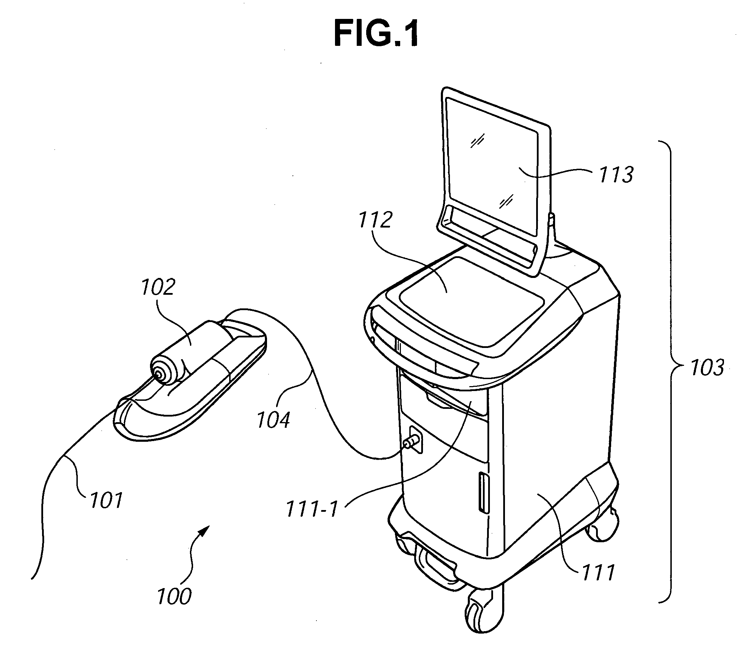

FIG. 1 is a diagram showing an external-appearance constitution of an optical imaging diagnostic apparatus (optical coherent tomography apparatus or optical frequency domain imaging apparatus utilizing wavelength sweep) 100 relating to a first exemplified embodiment of the present invention.

As shown in FIG. 1, the optical imaging diagnostic apparatus 100 is provided with an optical probe unit 101, a scanner & pull-back unit 102 and a steering control apparatus 103, and the scanner & pull-back unit 102 and the steering control apparatus 103 are connected by a signal line 104.

The optical probe unit 101 is inserted directly inside a body lumen of a blood vessel or the like and transmits the transmitted measurement light continuously to a biological tissue and concurrently, includes an imaging core provided with a transmitting and receiving unit for receiving the reflected light from the biological tissue continuously and a state of the biological tissue is measured by using the imaging core. The scanner & pull-back unit 102 is constituted with the optical probe unit 101 attachably and detachably and defines a radial operation of the imaging core inserted in the optical probe unit 101 depending on a fact that an installed motor is driven.

The steering control apparatus 103 includes, on an occasion when carrying out measurement, a function for inputting various kinds of setting values, and a function for processing data obtained by the measurement and for displaying it as a blood vessel cross-sectional image.

In the steering control apparatus 103, a reference numeral 111 indicates a main body control unit and it happens that the data obtained by the measurement is processed and the processed result is outputted. A reference numeral 111-1 indicates a printer & DVD recorder and it happens that the processed result in the main body control unit 111 is printed and is stored as data signals.

A reference numeral 112 indicates an operation panel and a user carries out inputs of various kinds of setting values and instruction through the operation panel 112. A reference numeral 113 indicates an LCD monitor as a display apparatus and it displays the processed result in the main body control unit 111.

### 2. Functional Constitution of Optical Coherent Tomography Apparatus

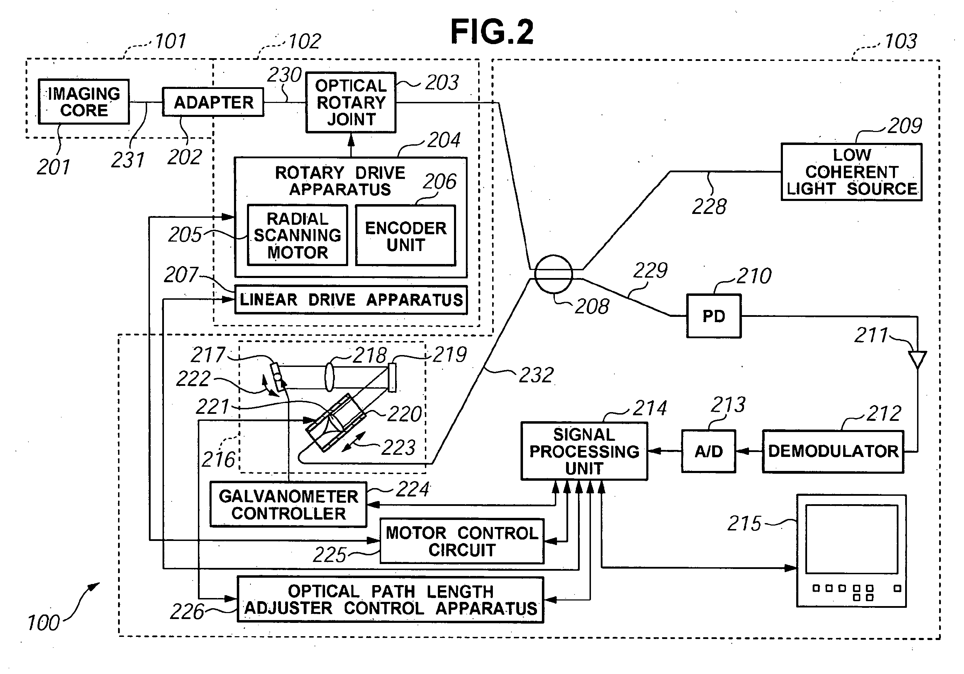

Next, it will be explained by using FIG. 2 with respect to a functional constitution of an optical coherent tomography apparatus within the optical imaging diagnostic apparatus 100 relating to this exemplified embodiment.

A 209 is a low coherent light source of a super high intensity light-emitting diode or the like. The low coherent light source 209 outputs a low coherent light whose wavelength is around 1310 nm and which shows coherence only in such a short distance range in which a coherent-able distance thereof (coherent length) is around a few µm to ten and a few µm.

Consequently, in a case in which this light is divided into two lights and thereafter, they are mixed again, the light is to be detected as a coherent light in a case in which the difference between the two optical path lengths from a point at which the light is divided to a point at which they are mixed is within a short distance range of around a few µm to ten and a few µm, and the light is never to be detected as a coherent light in a case in which the difference of the optical path lengths is larger than that.

The light of the low coherent light source 209 is entered into one end of a first single mode fiber 228 and is transmitted to the distal end surface side. The first single mode fiber 228 is connected optically with a second single mode fiber 229 and a third single mode fiber 232 by a photo coupler unit 208 on the way.

The photo coupler unit means an optical component in which it is possible to divide one optical signal into two or more outputs and/or to combine inputted two or more optical signals for one output and the light of the low coherent light source 209 is transmitted by being divided into three optical paths at the maximum by the photo coupler unit 208.

The scanner & pull-back unit 102 is provided on the distal end side ahead the photo coupler unit 208 of the first single mode fiber 228. In the inside of a rotary drive apparatus 204 of the scanner & pull-back unit 102, there is provided an optical rotary joint 203 for connecting between a non-rotary portion (fixed portion) and a rotary portion (rotary drive portion) and for transmitting the light.

Further, the distal end side of a fourth single mode fiber 230 on the inside of the optical rotary joint 203 is connected freely detachably with a fifth single mode fiber 231 of the optical probe unit 101 through an adapter 202. Thus, the light from the low coherent light source 209 is transmitted to the fifth single mode fiber 231 which is passed-through in the imaging core 201 provided with the transmitting and receiving unit, which repeats the transmitting and receiving of the light and which is rotary-drivable.

The light transmitted to the fifth single mode fiber 231 is emitted while radially operating with respect to the biological tissue inside the blood vessel from the transmitting and receiving unit arranged at the distal end of the imaging core 201. Then, a portion of the reflected light scattered on the surface or on the inside of the biological tissue is takenin by the imaging core 201 and returns to the first single mode fiber 228 side through a reverse optical path, and a portion thereof moves to the second single mode fiber 229 side by the photo coupler unit 208. Then, it is light-received by a photo detector (for example, photo diode 210) owing to a fact that it is emitted from one end of the second single mode fiber 229.

It should be noted that the rotary drive portion side of the optical rotary joint 203 is rotatingly driven by a radial scanning motor 205 of the rotary drive apparatus 204. Also, a rotary angle of the radial scanning motor 205 is detected by an encoder unit 206. Further, the scanner & pull-back unit 102 is provided with a linear drive apparatus 207 and movement (axial-direction operation) in an axial direction (distal direction of the body lumen and opposite direction thereof) of the imaging core 201 is defined based on an instruction from a signal processing unit 214. The axial-direction operation is realized owing to a fact that the linear drive apparatus 207 makes the scanner including the optical rotary joint 203 move based on a control signal from the signal processing unit 214.

At that time, only the imaging core 201 inserted into a catheter sheath moves axially while the catheter sheath of the optical probe unit 101 is maintained to be fixed in the blood vessel, so that it is possible to carry out the axial-direction operation without injuring a blood vessel wall.

On the other hand, a variable mechanism 216 of the optical path length for changing the optical path length of the reference light is provided on the distal end side ahead the photo coupler unit 208 of the third single mode fiber 232 (on the reference light path).

The variable mechanism 216 of this optical path length is provided with a first optical path length changing unit for changing the optical path length which corresponds to an inspection region in the depth direction (direction of emission of the measurement light) of the biological tissue speedily and a second optical path length changing unit for changing the optical path length which corresponds to fluctuation of the length thereof such that there can be absorbed the fluctuation of the length of the individual optical probe unit 101 in case of using the optical probe unit 101 by being exchanged.

Further, in the variable mechanism 216, facing the distal end of the third single mode fiber 232, there is arranged, through a collimating lens 221 which is freely movable in the direction shown in an arrow 223, with a mirror 219 which is mounted on an one-axis stage 220 together with this distal end. Also, there is mounted, through a mirror 218 corresponding to this mirror 219 (diffraction lattice), with a galvanometer 217 which is rotatable by a fine angle as the first optical path length changing unit. This galvanometer 217 is rotated speedily in an arrow 222 direction by a galvanometer controller 224.

The galvanometer 217 is a device which reflects light by a mirror of the galvanometer and it is constituted such that the mirror mounted on a movable portion thereof is to be rotated speedily by applying an AC drive signal to the galvanometer which functions as a reference mirror.

That is to say, owing to a fact that the drive signal is applied with respect to the galvanometer 217 from the galvanometer controller 224 and it is rotated speedily by the drive signal in the arrow 222 direction, it happens that the optical path length of the reference light is to change speedily only by the optical path length which corresponds to the inspection region in the depth direction of the biological tissue. One cycle of the change of this optical path difference becomes a cycle of obtaining coherent light for one line.

On the other hand, the one-axis stage 220 functions as the second optical path length changing unit having such an amount of variable range of optical path length, which can absorb the fluctuation of the optical path length of the optical probe unit 101 in case of exchanging the optical probe unit 101. Further, the one-axis stage 220 is also provided with a function as an adjuster for adjusting an offset. For example, even in a case in which the distal end of the optical probe unit 101 is not closely-attached to the surface of the biological tissue, it is possible, by changing the optical path length by the one-axis stage 220, to set it in a state of interfering with the reflected light from the surface position of the biological tissue.

The light whose optical path length is changed by the variable mechanism 216 of the optical path length is mixed with the reflected light obtained from the first single mode fiber 228 side by the photo coupler unit 208 which is provided on the way of the third single mode fiber 232 and is light-received as coherent light by the photo diode 210.

The coherent light which is light-received by the photo diode 210 in this manner is photoelectrically converted and amplified by an amplifier 211 and thereafter, is inputted to a demodulator 212.

In the demodulator 212, a demodulation process for extracting only the signal component of the coherent light is carried out and the output thereof is inputted to an A/D converter 213.

In the A/D converter 213, there is produced digital data "coherent light data" of one line by sampling the coherent light signal, for example, for 200 points. In this case, the sampling frequency becomes a value dividing one scanning time period of the optical path length by 200.

The coherent light data per line unit which is produced by the A/D converter 213 is inputted to a signal processing unit 214. In the signal processing unit 214, by carrying out a predetermined process with respect to the coherent light data, there is produced line data which are the coherent light intensity data in the depth direction of the biological tissue and thereafter, by converting the line data to the video signal, there is produced a cross-sectional image at each position in the axial direction inside the blood vessel, and it is outputted to a user interface apparatus 215 by a predetermined frame rate (corresponding to LCD monitor 113 and operation panel 112 in FIG. 1).

The signal processing unit 214 is connected further to an optical path length adjuster control apparatus 226. The signal processing unit 214 carries out the control of the position of the one-axis stage 220 through the optical path length adjuster control apparatus 226. Also, the signal processing unit 214 is connected to a motor control circuit 225 and controls the rotary drive of the radial scanning motor 205.

Further, the signal processing unit 214 is connected to the galvanometer controller 224 for controlling the scan of the optical path length of the reference mirror (galvanometer mirror) and the galvanometer controller 224 outputs the drive signal to the signal processing unit 214. In the motor control circuit 225, synchronization with the galvanometer controller 224 is taken by using this drive signal.

### 3. Functional Constitution of Optical Frequency Domain Imaging Apparatus Utilizing Wavelength Sweep

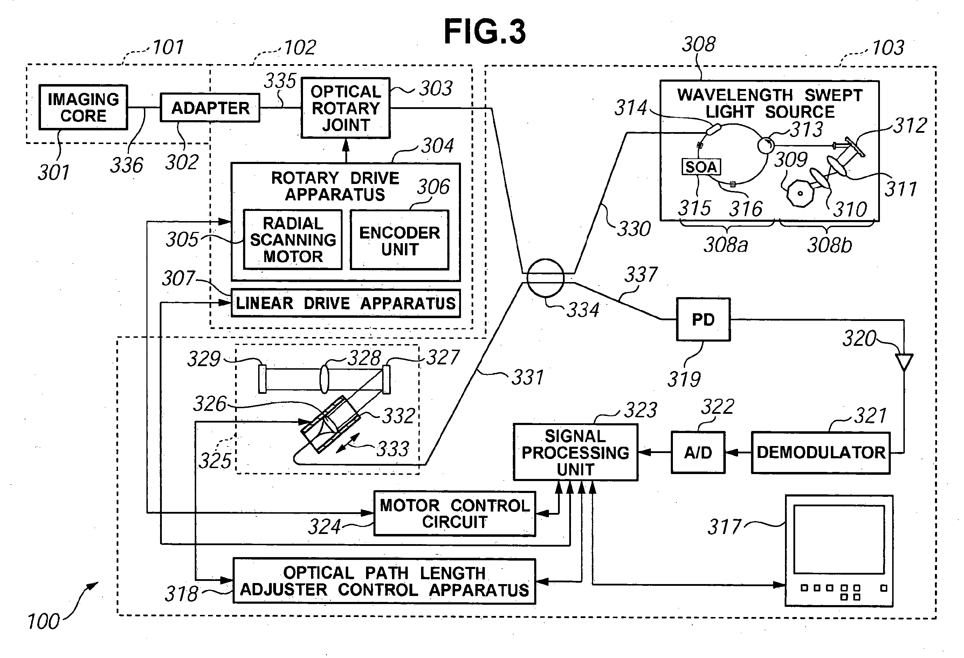

Next, it will be explained by using FIG. 3 with respect to a functional constitution of an optical frequency domain imaging apparatus utilizing wavelength sweep within the optical imaging diagnostic apparatus 100 relating to this exemplified embodiment.

FIG. 3 is a diagram showing a functional constitution of the optical frequency domain imaging apparatus 100 utilizing wavelength sweep.

A reference numeral 308 indicates a wavelength swept light source and a Swept-Laser is used. The wavelength swept light source 308 using the Swept-Laser is one kind of an Extended-Cavity-Laser which is composed of an optical fiber 316 connected with SOA315 (semiconductor optical amplifier) in a ring shape and a polygon scanning filter (308b).

The light outputted from the SOA315 proceeds inside the optical fiber 316 and enters in the polygon scanning filter 308b and the wavelength selected here is amplified by the SOA315 and finally, it is outputted from a coupler 314.

In the polygon scanning filter 308b, the wavelength is selected depending on the combination of a diffraction lattice 312 for light-splitting the light and a polygon mirror 309. Specifically, the light which is light-split by the diffraction lattice 312 is focused on the surface of the polygon mirror 309 depending on two pieces of lenses (310, 311). Thus, it happens that only the light of the wavelength perpendicular to the polygon mirror 309 returns to the same optical path and is outputted from the polygon scanning filter 308b, so that it is possible to carry out the time sweep of the wavelength by rotating the polygon mirror 309.

For the polygon mirror 309, for example, an icosadodecahedron mirror is used and the rotation speed thereof is around 50000 rpm. Owing to the wavelength sweep system in which the polygon mirror 309 and the diffraction lattice 312 are combined, it becomes possible to employ the wavelength sweep of high speed and high power.

The light of the wavelength swept light source 308, which is outputted from a coupler 314, is entered into one end of a first single mode fiber 330 and is transmitted to the distal end side. The first single mode fiber 330 is optically connected with a second single mode fiber 337 and a third single mode fiber 331 in a photo coupler unit 334 on the way. Therefore, the light which is entered into the first single mode fiber 330 is transmitted by being split into three optical paths at the maximum by this photo coupler unit 334.

On the distal end side ahead the photo coupler unit 334 of the first single mode fiber 330, there is provided, in a rotary drive apparatus 304, with an optical rotary joint 303 which connects between a non-rotary portion (fixed portion) and a rotary portion (rotary drive portion) and which transmits the light.

Further, the distal end side of a fourth single mode fiber 335 on the inside of the optical rotary joint 303 is connected freely detachably through a fifth single mode fiber 336 and an adapter 302 of the optical probe unit 101. Consequently, the light from the wavelength swept light source 308 is transmitted to the fifth single mode fiber 336 which is passed-through in an imaging core 301 and which is rotary-drivable.

The transmitted light is emitted from the transmitting and receiving unit arranged at a distal end of the imaging core 301 with respect to the biological tissue while being radially operated. Then, a portion of the reflected light which is scattered on the surface of or on the inside of the biological tissue is taken-in by the imaging core 301 and returns to the first single mode fiber 330 side through the reverse optical path. Further, the light is light-received by a photo detector (for example, photo diode 319) owing to a fact that a portion thereof moves to the second single mode fiber 337 side by the photo coupler unit 334 and is emitted from one end of the second single mode fiber 337.

It should be noted that the rotary drive portion side of the optical rotary joint 303 is rotatingly driven by a radial scanning motor 305 of the rotary drive apparatus 304. Also, the rotary angle of the radial scanning motor 305 is detected by an encoder unit 306. Further, the scanner & pull-back unit 102 is provided with a linear drive apparatus 307 and defines the axial-direction operation of the imaging core 301 based on an instruction from a signal processing unit 323.

On the other hand, there is provided a variable mechanism 325 of the optical path length for fine-adjusting the optical path length of the reference light at a distal end on the opposite side with respect to the photo coupler unit 334 of the third single mode fiber 331.

The variable mechanism 325 of this optical path length is provided with the optical path length changing unit for changing the optical path length which corresponds to the fluctuation of the length thereof such that the fluctuation of the length of the individual optical probe unit 101 can be absorbed in case of using the optical probe unit 101 by being exchanged.

The third single mode fiber 331 and a collimating lens 326 are provided on a one-axis stage 332 which is freely movable in the optical axial direction thereof as shown by an arrow 333, and they form the optical path length changing unit.

Specifically, the one-axis stage 332 functions as the optical path length changing unit having such an amount of variable range of optical path length, which can absorb the fluctuation of the optical path length of the optical probe unit 101 in case of exchanging the optical probe unit 101. Further, the one-axis stage 332 is also provided with a function as an adjuster for adjusting an offset. For example, even in a case in which the distal end of the optical probe unit 101 is not closely-attached to the surface of the biological tissue, it becomes possible, by changing the optical path length by the one-axis stage, to set it in a state of interfering with the reflected light from the surface position of the biological tissue.

The light whose optical path length is fine-adjusted by the variable mechanism 325 of the optical path length is mixed with the reflected light obtained from the first single mode fiber 330 side by the photo coupler unit 334 which is provided on the way of the third single mode fiber 331 and it is light-received by the photo diode 319.

The coherent light which is light-received by the photo diode 319 in this manner is photoelectrically converted and amplified by an amplifier 320 and thereafter, is inputted to a demodulator 321. In the demodulator 321, a demodulation process for extracting only the signal component of the coherent light is carried out and the output thereof is inputted to an A/D converter 322 as the coherent light signal.

In the A/D converter 322, there is produced digital data "coherent light data" of one line by sampling the coherent light signal, for example, for 2048 points by 180 MHz. It should be noted that the reason why the sampling frequency is set to be 180 MHz is because it is on the assumption that about 90% of the cycle (12.5µsec) of the wavelength sweep is to be extracted as the digital data of 2048 points in case of setting the repeat frequency of the wavelength sweep to be 80 kHz and it is not especially limited by this aspect.

The coherent light data per line unit produced in the A/D converter 322 is inputted to the signal processing unit 323. In the signal processing unit 323, the coherent light data are frequency-decomposed depending on a FFT (Fast Fourier Transform) and then, there are generated line data which are the coherent light intensity data in the depth direction, and by coordinate-converting those data, there is formed a cross-sectional image at each position in the axial direction of the inside of the blood vessel and it is outputted to an user interface apparatus 317 (which corresponds to LCD monitor 113 and operation panel 112 in FIG. 1) by a predetermined frame rate.

The signal processing unit 323 is connected further to an optical path length adjuster control apparatus 318. The signal processing unit 323 carries out the control of the position of the one-axis stage 332 through the optical path length adjuster control apparatus 318. Also, the signal processing unit 323 is connected to a motor control circuit 324 and receives a video synchronization signal of the motor control circuit 324. In the signal processing unit 323, the generation of the cross-sectional image is carried out in synchronization with the received video synchronization signal.

In addition, the video synchronization signal of this motor control circuit 324 is transmitted also to the rotary drive apparatus 304 and in the rotary drive apparatus 304, the drive signal in synchronization with the video synchronization signal is outputted.

### 4. Constitution of Distal End of Imaging Core Inserted in Optical Probe Unit and Operation of the Imaging Core

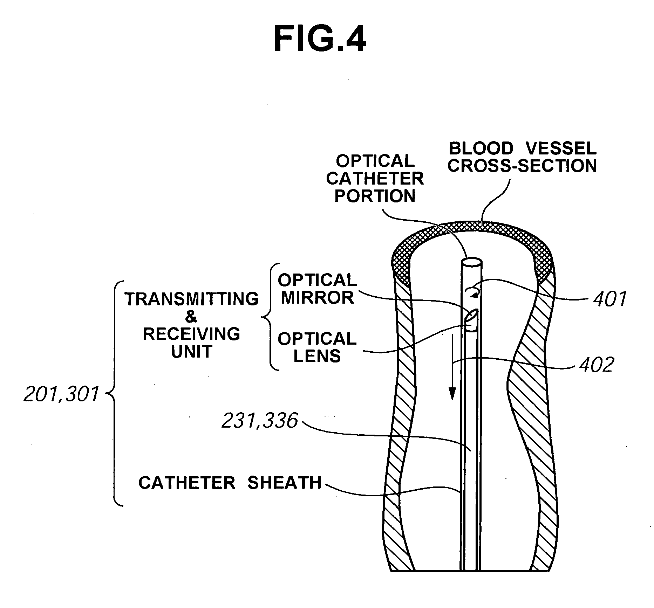

Next, it will be explained with respect to a constitution of a distal end of the imaging core 201, 301 inserted in the optical probe unit 101 and an operation of the imaging core 201, 301. First, it will be explained by using FIG. 4 with respect to a constitution of a distal end of the imaging core 201, 301 inserted in the optical probe unit 101. FIG. 4 is a diagram showing a state in which the optical probe unit 101 is inserted into a blood vessel.

As shown in FIG. 4, the imaging core 201, 301 provided with the transmitting and receiving unit and the optical fiber 231, 336 is inserted in the optical probe unit 101, and it is constituted so as to operate linearly in an arrow 402 direction while rotating in an arrow 401 direction inside the optical probe unit 101 which is inserted into the blood vessel (it is constituted so as to carry out radial operation).

The transmitting and receiving unit is provided with an optical mirror and an optical lens and emits the measurement light which is transmitted through the optical fibers 231, 336 in the direction approximately perpendicular to the axial direction (radial direction of the blood vessel). Also, the reflected light from the biological tissue with respect to the emitted measurement light is light-received and is transmitted to the steering control apparatus 103 side through the optical fibers 231, 336. Owing to a fact that the transmitting and receiving of such a light depending on the optical mirror and the optical lens is carried out during the radial operation of the imaging core 201, 301, it is possible to generate the line data for plural-generating the cross-sectional image inside the blood vessel in the axial direction.

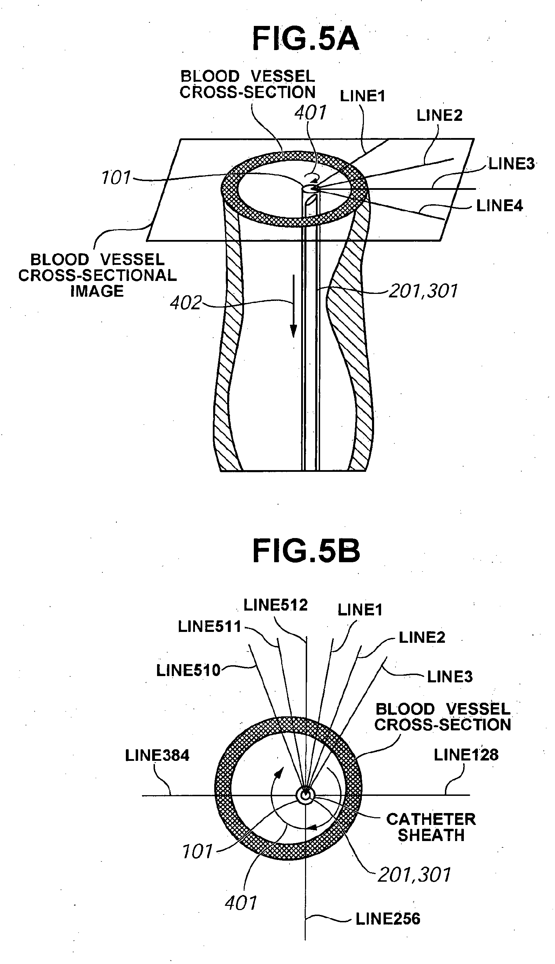

FIG. 5A is a diagram showing a state in which the measurement light is emitted toward the radial direction of the blood vessel while radially operating the imaging core 201, 301. As shown in FIG. 5A, owing to a fact that the measurement light is emitted while rotating the imaging core 201, 301 in the arrow 401 direction, it is possible to light-receive the reflected light from the biological tissue at each position direction of the circumference of the transmitting and receiving unit (that is, line data at each position can be generated). "Line 1", "Line 2", ... shown in FIG. 5A express the distribution of the data which are generated based on the reflected light from the biological tissue of the measurement light which is emitted with respect to the biological tissue at each position in the circumferential direction. In this manner, owing to the fact that the line data of a plurality of lines are generated at each position toward the circumferential direction in the axial direction, it is possible to generate the blood vessel cross-sectional image at each position in the axial direction.

FIG. 5B is a diagram showing one example of the number of times of the measurement-light emissions toward the radial direction of the blood vessel at a predetermined position in the axial direction. In the example of FIG. 5B, 512-times emissions are carried out while the imaging core 201, 301 is rotated once in the circumferential direction and the line data for 512 lines are generated.

### 5. Functional Constitution of Signal Processing Unit

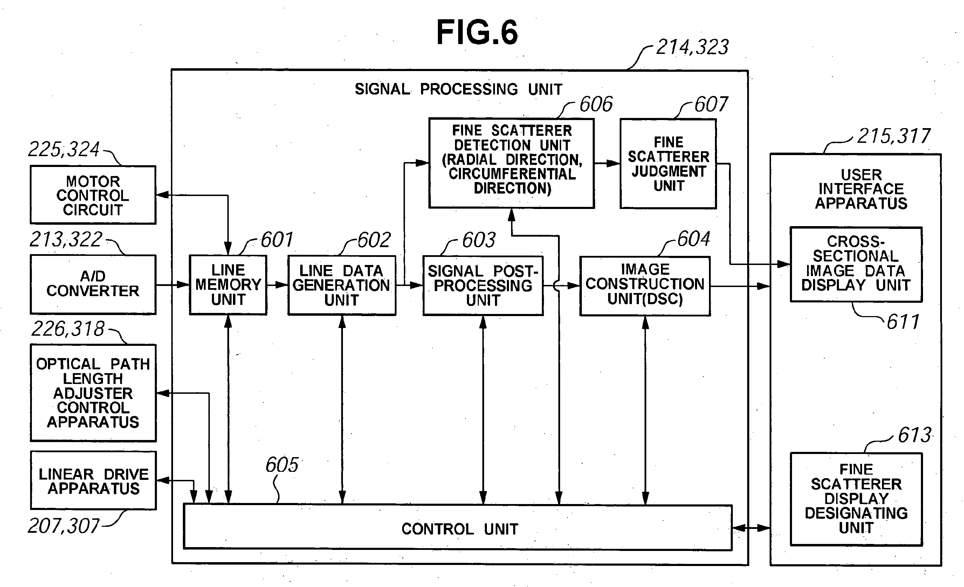

Next, by using FIG. 6, it will be explained, in the signal processing unit 214, 323 of the optical imaging diagnostic apparatus 100, with respect to a functional constitution of a drawing-out process for drawing out the blood vessel cross-sectional image and a minute scatterer detection process for clearly indicating the minute scatterer in the blood vessel cross-sectional image. It should be noted that it is allowed for the drawing-out process and the minute scatterer detection process explained hereinafter to be realized by using a hardware for exclusive use or it is also allowed for the function of each portion to be realized by a software (by executing a computer program).

FIG. 6 is a diagram showing a functional constitution of a drawing-out process and a minute scatterer detection process in the signal processing unit 214, 323 of the imaging diagnostic apparatus 100 and showing functional blocks related thereto. It should be noted in the following that in order to simplify the explanation, it is determined that it will be explained with respect to the signal processing unit 214 of the optical coherent tomography apparatus 100 (in FIG. 2) (in case of using an optical frequency domain imaging apparatus utilizing the wavelength sweep, similar functional blocks are also used, so that the explanation will be omitted here).

The coherent light data produced by the A/D converter 213 is processed in the line memory unit 601 such that the number of lines per one rotation of the radial scanning motor becomes 512 lines by using the signal of the encoder unit 206 of the radial scanning motor 205, which is outputted from the motor control circuit 225 and thereafter, it is outputted to the line data generation unit 602 in the succeeding stage.

In the line data generation unit 602, a line addition-averaging process, a filtering process, a logarithmic conversion process and the like are applied with respect to the coherent light data and the line data which is the coherent light intensity data in the depth direction of the biological tissue is generated and thereafter, the generated line data are outputted to the signal post-processing unit 603 in the succeeding stage. In the signal post-processing unit 603, a contrast adjustment, a intensity adjustment, a gamma correction, a frame correlation, a sharpness process and the like are carried out with respect to the line data and it is outputted to the image construction unit (DSC) 604.

In the image construction unit 604, a blood vessel cross-sectional image is generated owing to a fact that the line data series of the polar coordinate are Rθ-converted and thereafter, it is converted to a video signal and the blood vessel cross-sectional image is displayed on the cross-sectional image data display unit 611 of the user interface apparatus 215. It should be noted, in this exemplified embodiment, that it is assumed to generate the blood vessel cross-sectional image from 512 lines as one example, but it is not to be limited by this number of lines.

Also, in the explanation described above, it is explained by assuming that the signal post-processing unit 603 directly processes the line data outputted from the line data generation unit 602, but the present invention is not limited by this aspect and it is also allowed to be constituted such that the line data outputted from the line data generation unit 602 is parallelly stored in a storage unit which is not-shown as a file form in association with predetermined patient attribute information and measurement condition information. In this case, in the signal post-processing unit 603, it happens that the process described above is carried out in addition to a fact that the line data are read-out from the storage unit based on the instruction of a user. It should be noted that it is allowed for the storage unit to be provided inside the control unit 605 or to be provided outside the signal processing unit 214 (for example, it is also allowed for the DVD recorder 111-1 to function as the storage unit). Alternatively, it is also allowed for the line data generation unit 602 itself to have a function as the storage unit.

On the other hand, there is provided in the user interface apparatus 215 with a minute scatterer display instruction unit 613 and depending on this aspect, it is possible for a user to instruct so as to clearly indicate the existence of the minute scatterer on the blood vessel cross-sectional image which is displayed on the cross-sectional image data display unit 611.

When an instruction by the minute scatterer display instruction unit 613 is inputted, the instruction is transmitted to the minute scatterer detection unit 606 through the control unit 605. In the minute scatterer detection unit 606, a plurality of the line data used for the generation of the blood vessel cross-sectional image displayed on the cross-sectional image data display unit 611 is read-out from the storage unit and after carrying out a noise rejection process with respect to the plurality of the line data, a differentiation process is carried out in the radial direction and in the circumferential direction (change of the signal intensity is calculated). Thus, for example, it is possible to detect the minute scatterer of 20µm or less.

A detection result in the minute scatterer detection unit 606 is transmitted to a minute scatterer judgment unit 607 and it is judged therein whether or not the minute scatterer exists. It should be noted in the minute scatterer judgment unit 607 that an intersection of a detection result obtained by the differentiation process in the radial direction and a detection result obtained by the differentiation process in the circumferential direction is judged to be the minute scatterer. However, it is also allowed to be constituted such that an union of a detection result obtained by the differentiation process in the radial direction and a detection result obtained by the differentiation process in the circumferential direction is judged to be the minute scatterer.

In the minute scatterer judgment unit 607, further, the corresponding position on the cross-sectional image is calculated based on the position on the line data, which is judged to be the minute scatterer and it is outputted to the cross-sectional image data display unit 611. Then, in the cross-sectional image data display unit 611, a predetermined marker is displayed at the calculated position on the cross-sectional image. It should be noted that it will be explained in detail hereinafter with respect to the minute scatterer detection process from the instruction input in the minute scatterer display instruction unit 613 until the marker is displayed at the position of the minute scatterer on the blood vessel cross-sectional image.

### 6. Flow of Minute Scatterer Detection Process

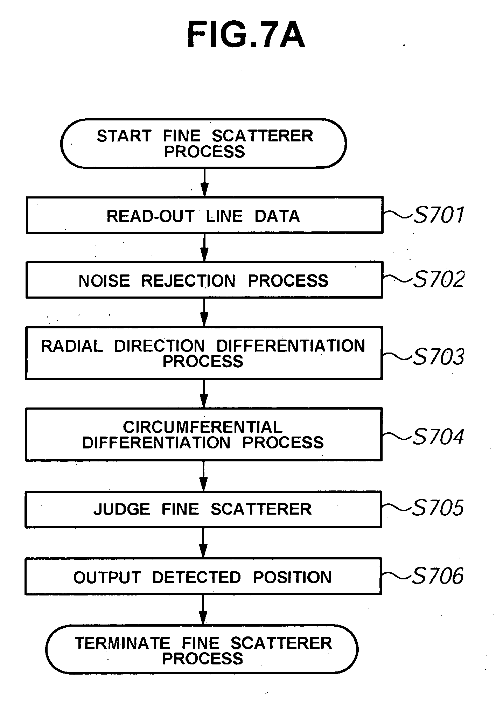

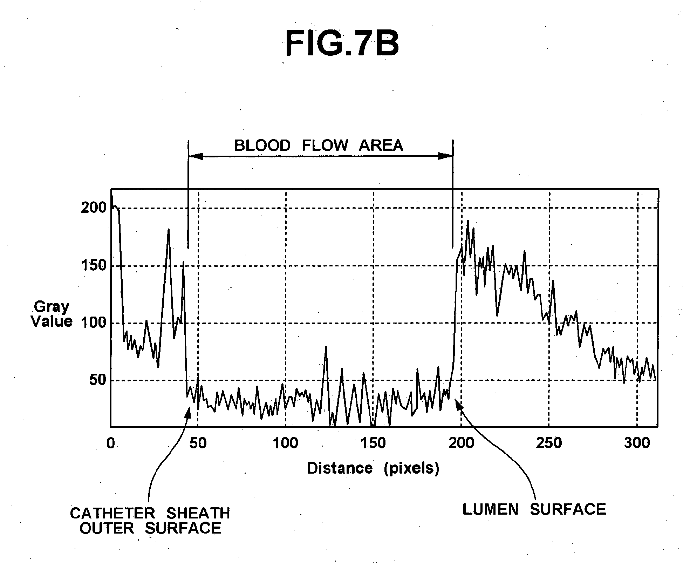

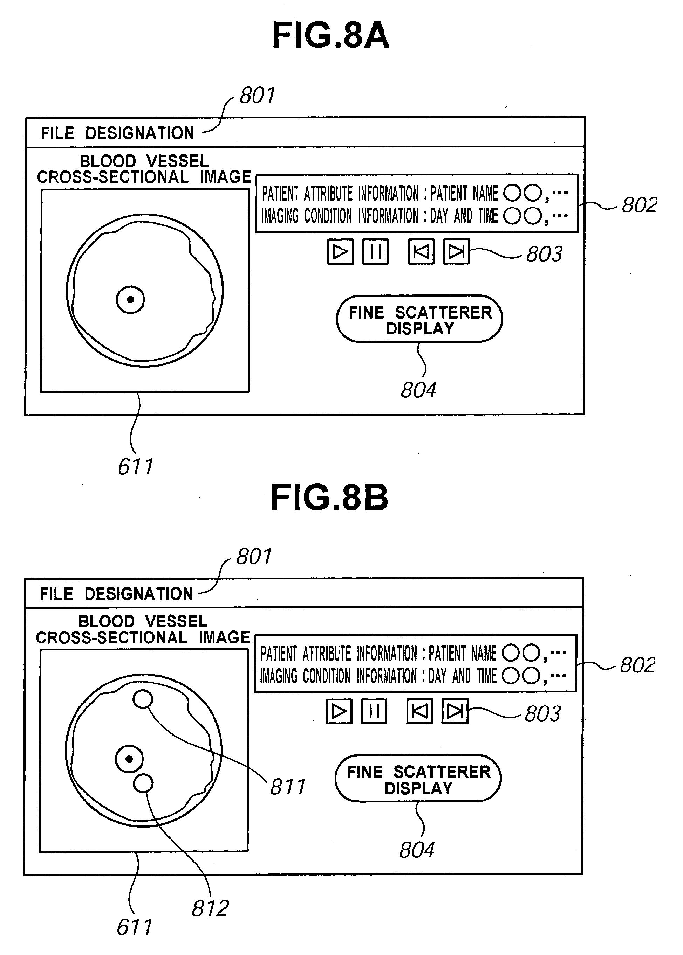

It will be explained by using FIG. 7A, FIG. 7B and FIGS. 8 with respect to the minute scatterer detection process. FIG. 7A is a flowchart showing a flow of the minute scatterer detection process for detecting the minute scatterer in the displayed blood vessel cross-sectional image and for displaying a marker thereof. Also, FIG. 7B is a diagram showing one example of the blood flow area on the line data. Further, FIGS. 8 are diagrams showing one example of the user interface for carrying out the minute scatterer detection process with respect to the displayed blood vessel cross-sectional image.

As shown in FIG. 8A, in the user interface for carrying out the minute scatterer detection process, there is provided a file-designation button 801 for designating a desired file from a plurality of files in which a plurality of line data used for the generation of the blood vessel cross-sectional image are stored. Also, there are provided a cross-sectional image data display unit 611 for displaying the blood vessel cross-sectional image continuously in real time at the time of measurement(or for displaying the blood vessel cross-sectional image continuously based on a plurality of the line data stored in the designated file) and a display area 802 for displaying various kinds of information (patient attribute information showing the attribute of a patient of a patient name and the like, and measurement condition information of a measurement day and time, the settings at the time of measurement and the like) with respect to the blood vessel cross-sectional image which is displayed on the cross-sectional image data display unit 611.

Further, in the cross-sectional image data display unit 611, there is provided an operation switch 803 for displaying a plurality of blood vessel cross-sectional images continuously, for temporarily-stopping the blood vessel cross-sectional image which is displayed continuously, for fast-forwarding or rewinding the display and the like.

Under such a constitution, a user operates, for example, the file-designation button 801 and reads-out a desired file and concurrently, makes the blood vessel cross-sectional image of the desired position in the axial direction be displayed on the cross-sectional image data display unit 611 by operating an operation switch 803. When the operation is completed by a user, the minute scatterer detection process shown in FIG. 7A is started.

When the minute scatterer detection process starts, in step S701, the minute scatterer detection unit 606 reads-out the plurality of line data (line data for one sheet of the blood vessel cross-sectional image, for example, line data for 512 lines) which are used to the generation of the blood vessel cross-sectional image displayed on the cross-sectional image data display unit 611 from the storage unit which is not-shown.

In step S702, a movement averaging process is carried out with respect to the plurality of line data read-out in step S701 (for example, filtering process is carried out by a five-point median filter) and the influence of the speckle noise is removed.

In step S703, the differentiation processes are carried out in the radial direction respectively with respect to all the plurality of line data in which the influence of the speckle noise is removed. By the differentiation processes, the minute scatterer is detected. Specifically, the differentiation filter is applied with respect to the respective line data, the fluctuation of the intensity value is calculated and the fluctuation in which the calculated result becomes a predetermined threshold value or more is detected. This process is carried out with respect to all the plurality of line data. Thus, for example, it is possible to detect the minute scatterer of 20µm or less from the respective line data.

Similarly, in step S704, the differentiation process is carried out in the circumferential direction respectively with respect to the plurality of line data in which the speckle noise is removed and the minute scatterer is detected (details of the differentiation process is similar as those in step S703).

It should be noted that with respect to the area which is applied with the differentiation filter in steps S703 and S704, the blood flow area inside the blood vessel (region from the outside of the outer surface of the catheter sheath of the optical probe unit 101 to the lumen position of the blood vessel, that is, area through which the physiological salt solution flows in a case in which the blood is removed by the flashing) is a target (see one example of the blood flow area shown in FIG. 7B) .

In step S705, the minute scatterer judgment unit 607 judges the intersection of the detection results detected in step S703 and step S704 as the differential scatterer. However, the present invention is not limited by this aspect and it is also allowed so as to judge the union of the detection results detected in step S703 and step S704 as the differential scatterer.

In step S706, there is calculated the position on the blood vessel cross-sectional image corresponding to the position on the line data which are judged as the differential scatterer in step S705. Further, the calculated position of the blood vessel cross-sectional image is outputted to the cross-sectional image data display unit 611 and there is displayed, on the cross-sectional image data display unit 611, a predetermined marker at that position.

In FIG. 8B, reference numerals 811, 812 show one example of the marker which is displayed on the blood vessel cross-sectional image. It should be noted in the example of FIG. 8B that it is constituted such that the position of the minute scatterer is to be indicated clearly by a circle centering around the position on the blood vessel cross-sectional image of the minute scatterer, but the style of the marker is not limited by this style and it is also allowed so as to indicate the position clearly by another marker (arrow or the like).

In this manner, by clearly indicating the minute scatterer whose existence is difficult to be confirmed on the displayed blood vessel cross-sectional image and which becomes a cause of the attenuation of the measurement light on the blood vessel cross-sectional image, it becomes possible for a user to distinguish the attenuation of the measurement light caused by the minute scatterer and the attenuation of the measurement light by a cause other than the minute scatterer.

As being clear from the explanation above, there is employed, in the optical imaging diagnostic apparatus relating to this exemplified embodiment, a constitution in which it is possible for a user to instruct so as to clearly indicate the position of the minute scatterer on the displayed blood vessel cross-sectional image.

Also, in case of obtaining an instruction from a user, there is employed a constitution in which the minute scatterer is detected by reading-out the line data used for the generation of the blood vessel cross-sectional image and by applying the differentiation process thereon in the radial direction and in the circumferential direction. Further, there is employed a constitution in which the position on the blood vessel cross-sectional image of the detected minute scatterer is calculated and the marker is displayed at that position.

Thus, it becomes possible for a user to identify the presence or non-presence of the minute scatterer on the blood vessel cross-sectional image.

### [Second Exemplified Embodiment]

In the first exemplified embodiment described above, in a case in which the minute scatterer is detected, there is employed a constitution in which the position of the minute scatterer on the blood vessel cross-sectional image is calculated and the marker is displayed at that position, but the present invention is not limited by this aspect. For example, it is also allowed to employ a constitution in which the blood vessel cross-sectional image is corrected so as to display a backward area (that is, area in which the measurement light is attenuated by the minute scatterer) of that position by being emphasized. In below, it will be explained hereinafter with respect to this exemplified embodiment centering around the difference with respect to the first exemplified embodiment described above.

### 1. Functional Constitution of Signal Processing Unit

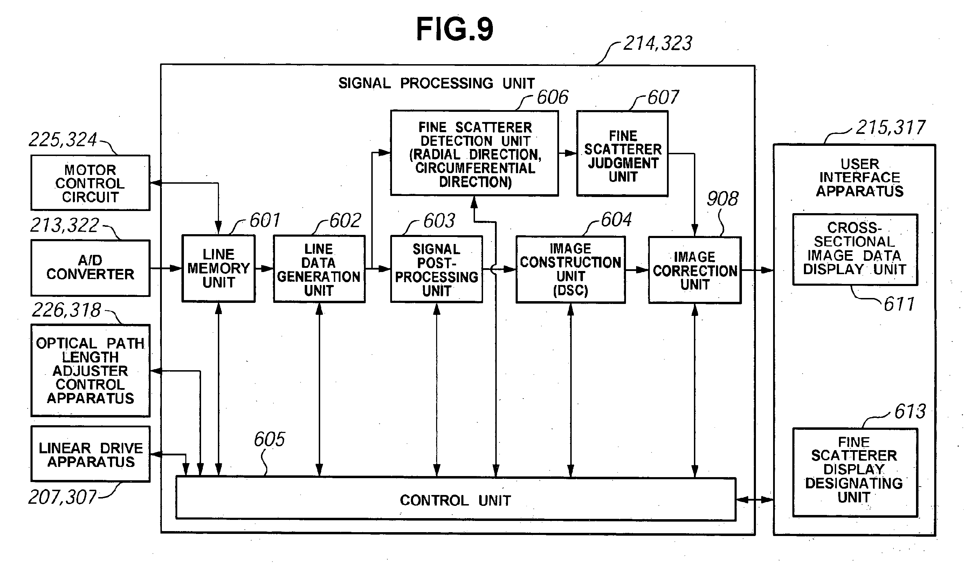

First, it will be explained by using FIG. 9 with respect to a functional constitution of a signal processing unit of this exemplified embodiment. It should be noted in FIG. 9 that the same reference numerals are assigned with respect to the same functional blocks as those of FIG. 6 and the explanations thereof will be omitted.

In FIG. 9, in the minute scatterer judgment unit 607, there is calculated the position on the blood vessel cross-sectional image based on the position on the line data which are judged as the minute scatterer and concurrently, the area on the cross-sectional image which corresponds to the backward area in the radial direction of that position is calculated. Further, with respect to the calculated backward area on the cross-sectional image, transmission to an image correction unit 908 is carried out.

In the image correction unit 908, each pixel on the cross-sectional image in the backward area is corrected and the backward area on the cross-sectional image is displayed by being emphasized.

### 2. Flow of Minute Scatterer Detection Process

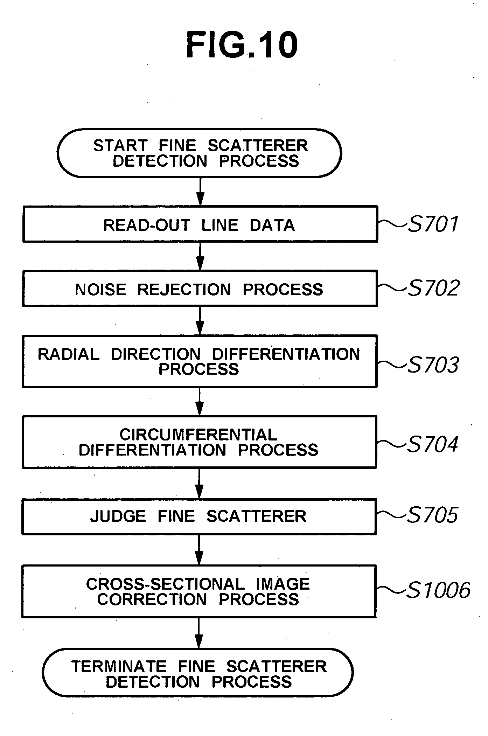

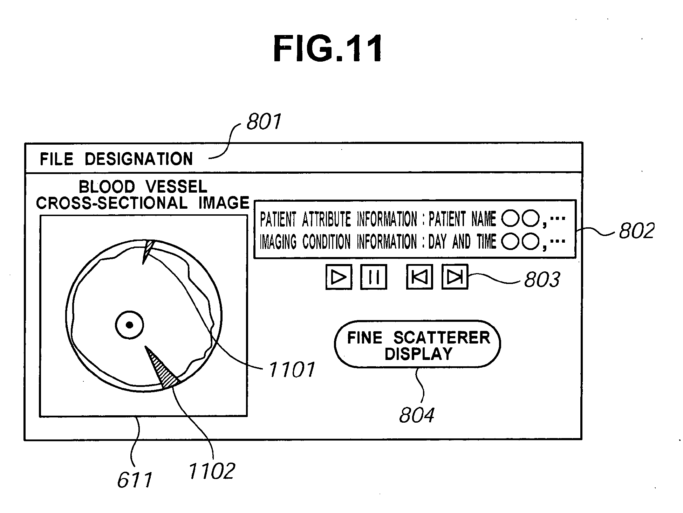

Next, it will be explained with respect to a minute scatterer detection process in this exemplified embodiment by using FIG. 10 and FIG. 11. FIG. 10 is a flowchart showing a flow of the minute scatterer detection process for detecting a minute scatterer in the displayed blood vessel cross-sectional image and for displaying a backward area of the detected minute scatterer by being emphasized. Also, FIG. 11 is a diagram showing one example of the user interface for carrying out the minute scatterer detection process with respect to the displayed blood vessel cross-sectional image.

It should be noted that the processes in steps S701 to S705 are the same as the processes in steps S701 to S705 explained in FIG. 7A, so that the explanations thereof will be omitted.

In step S1006, the minute scatterer judgment unit 607 specifies the backward area on the blood vessel cross-sectional image of the minute scatterer which is detected. Further, the image correction unit 908 corrects the blood vessel cross-sectional image with respect to the specified backward area. That is, the image correction unit 908 corrects the displayed cross-sectional image of the backward area to display each pixel of the backward area in the displayed cross-sectional image in an emphasized or highlighted manner to distinguish the pixels of the backward area in a visually distinguishing manner relative to the portion of the displayed cross-sectional image adjacent the backward area.

FIG. 11 is a diagram showing one example of the blood vessel cross-sectional image after carrying out the cross-sectional image correction process in step S1006. In FIG. 11, reference numerals 1101 and 1102 indicate areas which are displayed by being emphasized in the backward area of the minute scatterer which is detected.

In this manner, by employing a constitution in which the backward area of the minute scatterer is displayed by being emphasized, it becomes possible to make a user recognize on the blood vessel cross-sectional image that the aforementioned area is an area which received the influence of the attenuation of the measurement light caused by the minute scatterer.

### [Third Exemplified Embodiment]

In the second exemplified embodiment described above, there is employed a constitution in which by displaying the backward area of the detected minute scatterer by being emphasized on the blood vessel cross-sectional image, a user is made to recognize the aforementioned area is an area which received the influence of the attenuation of the measurement light caused by the minute scatterer, but the present invention is not limited by this aspect.

For example, with respect to the backward area which receives the influence of the attenuation of the measurement light caused by the minute scatterer, it is also allowed to employ a constitution in which the blood vessel cross-sectional image is corrected so as to remove the influence of the attenuation of the measurement light. Hereinafter, it will be explained with respect to details of this exemplified embodiment.

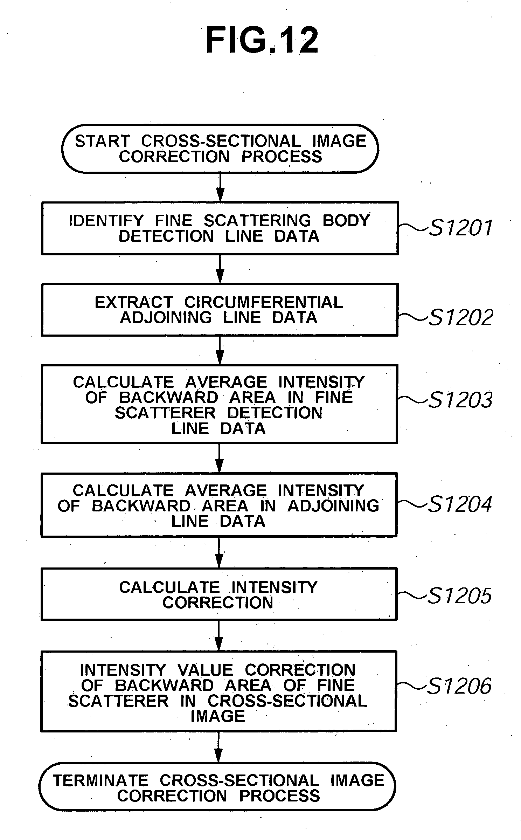

FIG. 12 is a flowchart showing a flow of the cross-sectional image correction process (step S1006 of FIG. 10) in this exemplified embodiment.

In step S1201, the minute scatterer judgment unit 607 identifies the line data (minute scatterer detection line data) in which the minute scatterer is detected in step S706.

In step S1202, there are extracted the line data (adjacent line data) which are adjacent with the minute scatterer detection line data identified in step S1201 in a circumferential direction (there are extracted line data which are adjacent with the minute scatterer detection line data in a circumferential direction on the blood vessel cross-sectional image).

In step S1203, there is calculated an average intensity in the backward area of the minute scatterer of the minute scatterer detection line data identified in step S1201. Also, in step S1204, there is calculated an average intensity in the backward area of the adjacent line data extracted in step S1202.

In step S1205, by comparing the average intensity calculated in step S1203 and the average intensity calculated in step S1204, there is calculated a intensity correction value in the backward area of the minute scatterer of the minute scatterer detection line data.

In step S1206, based on the intensity correction value calculated in step S1205, the image correction unit 908 corrects the intensity value of each pixel of the backward area of the minute scatterer in the blood vessel cross-sectional image.

As being clear from the explanation above, according to this exemplified embodiment, it becomes possible, with respect to the backward area which receives the influence of the attenuation of the measurement light caused by the minute scatterer, to remove the influence of the attenuation of the measurement light.

### [Fourth Exemplified Embodiment]

In the third exemplified embodiment described above, there is employed a constitution in which the intensity correction value in the backward area of the minute scatterer detection line data is calculated by using the adjacent line data, but the present invention is not limited by this aspect. For example, the degree of influence of the attenuation of the measurement light caused by the minute scatterer becomes different in response to the size of the minute scatterer, so that it is also allowed to employ a constitution in which the attenuation curve is preliminarily kept for every size of the minute scatterer and the intensity correction value is calculated by using the attenuation curve based on the detected size of the minute scatterer.

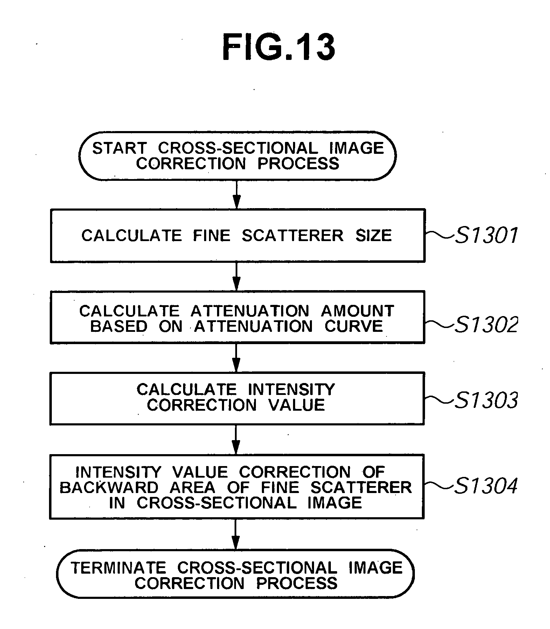

FIG. 13 is a flowchart showing a flow of the cross-sectional image correction process (step S1006 of FIG. 10) in this exemplified embodiment.

In step S1301, the minute scatterer judgment unit 607 calculates the size of the minute scatterer which is detected in step S706. In step S1302, there is extracted the attenuation curve corresponding to the size of the minute scatterer which is calculated in step S1301 (attenuation curve corresponding to the size of the minute scatterer, which is calculated, in step S1301, from within the attenuation curves kept preliminarily for every size of the minute scatterer) and the amount of attenuation at each position in the radial direction is calculated based on that extracted attenuation curve.

In step S1303, the intensity correction value is calculated based on the amount of attenuation which is calculated in step S1302.

In step S1304, the image correction unit 908 calculates the intensity value of each pixel of the backward area of the minute scatterer in the blood vessel cross-sectional image based on the intensity correction value which is calculated in step S1303.

As being clear from the explanation above, according to this exemplified embodiment, it becomes possible to remove the influence of the attenuation of the measurement light with respect to the backward area which receives the influence of the attenuation of the measurement light caused by the minute scatterer.

Having described preferred embodiments of the invention with reference to the accompanying drawings, it is to be understood that the invention is not limited to those precise embodiments and that various changes and modifications could be effected therein by one skilled in the art without departing from the scope of the invention as defined in the appended claims.

## Claims

1. An optical imaging diagnostic apparatus (100) connected to a probe (101), said optical imaging diagnostic apparatus (100) further including
a transmitting and receiving unit (201, 301) for carrying out transmitting and receiving of light continuously, adapted to obtain reflected light from a biological tissue, the transmitting and receiving unit being axially movable inside a body lumen while rotating, the apparatus being configured to generate a plurality of cross-sectional images of the biological tissue in the axial direction by using line data derived from coherent light produced by interference between the obtained reflected light and reference light, and to display the images thereof, **characterized in that** the optical imaging diagnostic apparatus (100) further comprises:
a calculation unit (605) for respectively calculating signal-intensity change in the circumferential direction of the circumference of the position of the transmitting and receiving unit (201, 301) and signal-intensity change in the radial direction of the body lumen from a plurality of line data used for generation of the cross-sectional image displayed; and a specifying unit (607) for judging, based on the calculated result in the calculation unit (605), whether or not there exist scatterers consisting of blood cells or floating thrombi, which scatter the light transmitted from the transmitting and receiving unit (201, 301) in an blood flow area inside the body lumen and for specifying the position of the scatterers on the displayed cross-sectional image of the scatterer in case of being judged that there exists the scatterers; and
a display unit (215, 317) for displaying a marker indicating that the scatterer exists at the specified position on the displayed cross-sectional image.

2. The optical imaging diagnostic apparatus according to claim 1, wherein the specifying unit (607) is configured to judge that the scatterer exists at a position within an area in which both the signal-intensity change in the circumferential direction as well as the signal change in the radial direction are equal or larger to predetermined threshold values..

3. The optical imaging diagnostic apparatus according to claim 1, further comprising, with respect to the specified position, a correction unit (908) for making correction about the displayed cross-sectional image of the backward area where the measurement light is attenuated due to the presence of minute scatterers which are positioned backward in the radial direction of the body lumen.

4. The optical imaging diagnostic apparatus according to claim 3, wherein the correction unit (908) is configured to make correction so as to display each pixel of the backward area in the displayed cross-sectional image by being emphasized.

5. The optical imaging diagnostic apparatus according to claim 3, wherein the correction unit (908) is configured to calculate a correction value by comparing signal intensity of line data which were judged such that the scatterers exist therein and signal intensity of line data which are neighboring in the circumferential direction with respect to the line data which were judged such that the scatterers exist therein, and corrects the intensity value of each pixel of the backward area in the displayed cross-sectional image depending on the calculated correction value.

6. The optical imaging diagnostic apparatus according to claim 3 further comprising:
a holding unit for preliminarily holding an attenuation curve indicating light attenuation caused by a scatterer for every size of the scatterers, wherein
the correction unit (908) is configured to calculate a correction value of the backward area in the displayed cross-sectional image based on the attenuation curve by calculating the size of the scatterers judged in the specifying unit (607), and corrects the intensity value of each pixel of the backward area in the displayed cross-sectional image depending on the calculated correction value.

7. A display control method in an optical imaging diagnostic apparatus (100) including the steps of:
connecting a probe (101) including a transmitting and receiving unit (201, 301) for carrying out transmitting and receiving of light continuously, obtaining reflected light from a biological tissue, which the transmitting and receiving unit received, from the transmitting and receiving unit by axially moving the transmitting and receiving unit inside a body lumen while rotating it, generating a plurality of cross-sectional images of the biological tissue in the axial direction by using line data derived from coherent light produced by interference between the obtained reflected light and reference light, and displaying the images thereof, **characterized in that** the display control method further comprises:
a calculating process for respectively calculating signal-intensity change in the circumferential direction of the circumference of the position of the transmitting and receiving unit and signal-intensity change in the radial direction of the body lumen respectively from a plurality of the line data used for generation of the cross-sectional image displayed;
a specifying process for judging, based on the calculated result in the calculating process, whether or not there exist scatterers consisting of blood cells or floating thrombi, which scatter the light transmitted from the transmitting and receiving unit in an blood flow area of the body lumen and for specifying the position of the scatterers on the displayed cross-sectional image of the scatterer in case of being judged that there exists the scatterer; and
a display process for displaying a marker indicating that the scatterer exists at the specified position on the displayed cross-sectional image.

## Patentansprüche

1. Optische Bildgebungsdiagnosevorrichtung (100), die mit einer Sonde (101) verbunden ist, wobei die optische Bildgebungsdiagnosevorrichtung (100) ferner Folgendes einschließt:
eine Sende- und Empfangseinheit (201, 301) zum kontinuierlichen Durchführen des Sendens und Empfangens von Licht, die dafür eingerichtet ist, reflektiertes Licht von einem biologischen Gewebe zu erfassen, wobei die Sende- und Empfangseinheit in Axialrichtung innerhalb eines Körperlumens beweglich ist, während sie sich dreht, wobei die Vorrichtung dafür konfiguriert ist, durch die Verwendung von Liniendaten, die von kohärentem Licht abgeleitet sind, das durch Interferenz zwischen dem erfassten reflektierten Licht und Referenzlicht erzeugt wird, mehrere Querschnittsbilder des biologischen Gewebes in der Axialrichtung zu erzeugen und die Bilder desselben anzuzeigen,
**dadurch gekennzeichnet, dass** die optische Bildgebungsdiagnosevorrichtung (100) ferner Folgendes umfasst:
eine Berechnungseinheit (605) zum jeweiligen Berechnen einer Signalintensitätsänderung in der Umfangsrichtung des Umfangs des Abschnitts der Sende- und Empfangseinheit (201, 301) beziehungsweise einer Signalintensitätsänderung in der Radialrichtung des Körperlumens aus mehreren Liniendaten, die zur Erzeugung des angezeigten Querschnittsbildes verwendet werden, und
eine Spezifizierungseinheit (607) zum Einschätzen, auf der Grundlage des in der Berechnungseinheit (605) berechneten Ergebnisses, ob es Streuer, die aus Blutzellen oder schwebenden Thromben bestehen, die das von der Sende- und Empfangseinheit (201, 301) gesendete Licht streuen, in einem Blutflussbereich innerhalb des Körperlumens gibt oder nicht, und zum Spezifizieren der Position der Steuer auf dem angezeigten Querschnittsbild des Streuers in dem Fall, dass eingeschätzt wird, dass es die Streuer gibt, und
eine Anzeigeeinheit (215, 317) zum Anzeigen einer Markierung, die angibt, dass es den Streuer an der spezifizierten Position auf dem angezeigten Querschnittsbild gibt.

2. Optische Bildgebungsdiagnosevorrichtung nach Anspruch 1, wobei die Spezifizierungseinheit (607) dafür konfiguriert ist, einzuschätzen, dass der Streuer an einer Position innerhalb eines Bereichs vorhanden ist, in dem sowohl die Signalintensitätsänderung in der Umfangsrichtung als auch die Signalintensitätsänderung in der Radialrichtung gleich vorbestimmten Schwellenwerten oder größer als dieselben sind.

3. Optische Bildgebungsdiagnosevorrichtung nach Anspruch 1, die ferner, in Bezug auf die spezifizierte Position, eine Korrektureinheit (908) zum Vornehmen einer Korrektur um das angezeigte Querschnittsbild des hinteren Bereichs, wo das Messlicht auf Grund des Vorhandenseins von winzigen Streuern, die in der Radialrichtung des Körperlumens nach hinten angeordnet sind, gedämpft wird, umfasst.

4. Optische Bildgebungsdiagnosevorrichtung nach Anspruch 3, wobei die Korrektureinheit (908) dafür konfiguriert ist, die Korrektur so vorzunehmen, dass jeder Bildpunkt des hinteren Bereichs in dem angezeigten Querschnittsbild dadurch angezeigt wird, dass er angehoben wird.

5. Optische Bildgebungsdiagnosevorrichtung nach Anspruch 3, wobei die Korrektureinheit (908) dafür konfiguriert ist, durch das Vergleichen der Signalintensität von Liniendaten, die derart eingeschätzt wurden, dass es in denselben die Streuer gibt, und der Signalintensität von Liniendaten, die in der Umfangsrichtung in Bezug auf die Liniendaten, die derart eingeschätzt wurden, dass es in denselben die Streuer gibt, benachbart sind, einen Korrekturwert zu berechnen, und den Intensitätswert jedes Bildpunkts des hinteren Bereichs in dem angezeigten Querschnittsbild in Abhängigkeit von dem berechneten Korrekturwert korrigiert.

6. Optische Bildgebungsdiagnosevorrichtung nach Anspruch 3, die ferner Folgendes umfasst:
eine Speichereinheit zum vorläufigen Speichern einer Dämpfungskurve, die eine durch einen Streuer verursachte Lichtdämpfung für jede Größe der Streuer angibt, wobei
die Korrektureinheit (908) dafür konfiguriert ist, auf der Grundlage der Dämpfungskurve durch das Berechnen der Größe der in der Spezifizierungseinheit (607) eingeschätzten Streuer einen Korrekturwert des hinteren Bereichs in dem angezeigten Querschnittsbild zu berechnen, und den Intensitätswert jedes Bildpunkts des hinteren Bereichs in dem angezeigten Querschnittsbild in Abhängigkeit von dem berechneten Korrekturwert korrigiert.

7. Anzeigesteuerungsverfahren in einer optischen Bildgebungsdiagnosevorrichtung (100), das die folgenden Schritte einschließt:
das Anschließen einer Sonde (101), die eine Sende- und Empfangseinheit (201, 301) zum kontinuierlichen Durchführen des Sendens und Empfangens von Licht einschließt, das Erfassen von reflektiertem Licht von einem biologischen Gewebe, das die Sende- und Empfangseinheit empfing, von der Sende- und Empfangseinheit durch das Bewegen der Sende- und Empfangseinheit in Axialrichtung innerhalb eines Körperlumens, während sie gedreht wird, wobei die Vorrichtung dafür konfiguriert ist, das Erzeugen mehrerer Querschnittsbilder des biologischen Gewebes in der Axialrichtung durch die Verwendung von Liniendaten, die von kohärentem Licht abgeleitet sind, das durch Interferenz zwischen dem erfassten reflektierten Licht und Referenzlicht erzeugt wird, und das Anzeigen der Bilder desselben,
**dadurch gekennzeichnet, dass** das Anzeigesteuerungsverfahren ferner Folgendes umfasst:
einen Berechnungsvorgang zum jeweiligen Berechnen einer Signalintensitätsänderung in der Umfangsrichtung des Umfangs des Abschnitts der Sende- und Empfangseinheit beziehungsweise einer Signalintensitätsänderung in der Radialrichtung des Körperlumens aus mehreren Liniendaten, die zur Erzeugung des angezeigten Querschnittsbildes verwendet werden,
einen Spezifizierungsvorgang zum Einschätzen, auf der Grundlage des in dem Berechnungsvorgang berechneten Ergebnisses, ob es Streuer, die aus Blutzellen oder schwebenden Thromben bestehen, die das von der Sende-und Empfangseinheit gesendete Licht streuen, in einem Blutflussbereich des Körperlumens gibt oder nicht, und zum Spezifizieren der Position der Steuer auf dem angezeigten Querschnittsbild des Streuers in dem Fall, dass eingeschätzt wird, dass es den Streuer gibt, und
einen Anzeigevorgang zum Anzeigen einer Markierung, die angibt, dass es den Streuer an der spezifizierten Position auf dem angezeigten Querschnittsbild gibt.

## Revendications

1. Appareil de diagnostic par imagerie optique (100) connecté à une sonde (101), ledit appareil de diagnostic par imagerie optique (100) comprenant en outre :
une unité d'émission et de réception (201, 301) pour réaliser l'émission et la réception de lumière de façon continue, adaptée pour obtenir une lumière réfléchie par un tissu biologique, l'unité d'émission et de réception étant mobile axialement à l'intérieur d'une lumière corporelle tout en la faisant tourner, l'appareil étant configuré pour générer une pluralité d'images en coupe transversale du tissu biologique dans la direction axiale en utilisant des données de ligne obtenues à partir d'une lumière cohérente produite par interférence entre la lumière réfléchie obtenue et une lumière de référence, et pour afficher les images de celui-ci ;
**caractérisé en ce que** l'appareil de diagnostic par imagerie optique (100) comprend en outre :
une unité de calcul (605) pour calculer respectivement une variation d'intensité de signal dans la direction circonférentielle de la circonférence de la position de l'unité d'émission et de réception (201, 301) et une variation d'intensité de signal dans la direction radiale de la lumière corporelle à partir d'une pluralité de données de ligne utilisées pour la génération de l'image en coupe transversale affichée ; et
une unité de spécification (607) pour déterminer, d'après le résultat calculé dans l'unité de calcul (605), s'il existe ou non des diffuseurs constitués par des cellules sanguines ou des thrombus flottants, qui diffusent la lumière transmise par l'unité d'émission et de réception (201, 301) dans une zone d'écoulement sanguin à l'intérieur de la lumière corporelle et pour spécifier la position des diffuseurs sur l'image du diffuseur en coupe transversale affichée dans le cas où il est déterminé qu'il existe des diffuseurs ; et
une unité d'affichage (215, 317) pour afficher un marqueur qui indique que le diffuseur existe à la position spécifiée sur l'image en coupe transversale affichée.

2. Appareil de diagnostic par imagerie optique selon la revendication 1, dans lequel l'unité de spécification (607) est configurée pour déterminer que le diffuseur existe en une position située dans une zone dans laquelle à la fois la variation d'intensité de signal dans la direction circonférentielle et la variation de signal dans la direction radiale sont supérieures ou égales à des valeurs de seuil prédéterminées.

3. Appareil de diagnostic par imagerie optique selon la revendication 1, comprenant en outre, relativement à la position spécifiée, une unité de correction (908) pour effectuer une correction sur l'image en coupe transversale affichée de la région située en arrière, où la lumière de mesure est atténuée en raison de la présence de minuscules diffuseurs qui sont positionnés en arrière dans la direction radiale de la lumière corporelle.

4. Appareil de diagnostic par imagerie optique selon la revendication 3, dans lequel l'unité de correction (908) est configurée pour réaliser une correction qui fait afficher chaque pixel de la région arrière en étant mis en valeur dans l'image en coupe transversale affichée.

5. Appareil de diagnostic par imagerie optique selon la revendication 3, dans lequel l'unité de correction (908) est configurée pour calculer une valeur de correction en comparant l'intensité de signal de données de ligne pour lesquelles il a été déterminé que des diffuseurs existent et l'intensité de signal de données de ligne qui sont voisines dans la direction circonférentielle par rapport aux données de ligne pour lesquelles il a été déterminé que des diffuseurs existent, et corrige la valeur d'intensité de chaque pixel de la région arrière dans l'image en coupe transversale affichée en fonction de la valeur de correction calculée.

6. Appareil de diagnostic par imagerie optique selon la revendication 3, comprenant en outre :
une unité de maintien pour maintenir de façon préliminaire une courbe d'atténuation indiquant une atténuation de lumière provoquée par un diffuseur pour toutes les tailles de diffuseurs, dans lequel
l'unité de correction (908) est configurée pour calculer une valeur de correction de la région arrière dans l'image en coupe transversale affichée basée sur la courbe d'atténuation en calculant la taille des diffuseurs évalués dans l'unité de spécification (607), et corrige la valeur d'intensité de chaque pixel de la région arrière dans l'image en coupe transversale affichée en fonction de la valeur de correction calculée.

7. Procédé de commande d'affichage dans un appareil de diagnostic par imagerie optique (100) comprenant les étapes suivantes :
connecter une sonde (101) comportant une unité d'émission et de réception (201, 301) pour réaliser l'émission et la réception de lumière de façon continue, obtenir une lumière réfléchie par un tissu biologique, qui a été reçue par l'unité d'émission et de réception, provenant de l'unité d'émission et de réception en déplaçant axialement l'unité d'émission et de réception à l'intérieur d'une lumière corporelle tout en la faisant tourner, générer une pluralité d'images en coupe transversale du tissu biologique dans la direction axiale en utilisant des données de ligne obtenues à partir d'une lumière cohérente produite par interférence entre la lumière réfléchie obtenue et une lumière de référence, et afficher les images de celui-ci,
**caractérisé en ce que** le procédé de commande d'affichage comprend en outre :
un processus de calcul pour calculer respectivement une variation d'intensité de signal dans la direction circonférentielle de la circonférence de la position de l'unité d'émission et de réception et une variation d'intensité de signal dans la direction radiale de la lumière corporelle à partir respectivement d'une pluralité des données de ligne utilisées pour la génération de l'image en coupe transversale affichée ;
un processus de spécification pour déterminer, d'après le résultat calculé dans le processus de calcul, s'il existe ou non des diffuseurs constitués par des cellules sanguines ou des thrombus flottants, qui diffusent la lumière transmise par l'unité d'émission et de réception dans une zone d'écoulement sanguin de la lumière corporelle et pour spécifier la position des diffuseurs sur l'image du diffuseur en coupe transversale affichée dans le cas où il est déterminé qu'il existe des diffuseurs ; et
un processus d'affichage pour afficher un marqueur qui indique que le diffuseur existe à la position spécifiée sur l'image en coupe transversale affichée.
